# Europäisches Patentamt
# European Patent Office
# Office européen des brevets

(19)

(11) Publication number: **0 030 110**
B1

(12)

# EUROPEAN PATENT SPECIFICATION

(45) Date of publication of patent specification: **25.07.84**

(21) Application number: **80304194.6**

(22) Date of filing: **21.11.80**

(51) Int. Cl.³: **C 07 C 29/15,**
**C 07 C 31/08,**
**C 07 C 45/49,**
**C 07 C 47/06,**
**C 07 C 53/08, C 07 C 51/10**
**//B01J23/46**

(54) Process for the production of an oxygenated hydrocarbon product containing ethanol.

(30) Priority: **27.11.79 GB 7940844**

(43) Date of publication of application:
**10.06.81 Bulletin 81/23**

(45) Publication of the grant of the patent:
**25.07.84 Bulletin 84/30**

(84) Designated Contracting States:
**BE DE FR GB IT NL**

(56) References cited:
EP - A - 0 004 656
EP - A - 0 010 295
EP - A - 0 022 358
FR - A - 2 234 256
FR - A - 2 317 260
BULLETIN OF THE CHEMICAL SOCIETY OF
JAPAN, vol. 51, no. 8, August 1978, Tokyo, JP
MASARU ICHIKAWA "Catalysis by supported
metal crystallites from carbonyl clusters. II.
Catalytic ethanol synthesis from Co and H2
under atmospheric pressure over supported
rhodium crystallites prepared from Rh carbonyl
clusters deposited on TiO2, ZeO2 and La2O3".
pages 2273-2277
JOURNAL OF THE CHEMICAL SOCIETY,
CHEMICAL COMMUNICATIONS July 5, 1978,
no. 13, MASARU ICHIKAWA "Catalytic

(73) Proprietor: **The British Petroleum Company p.l.c.**
**Britannic House Moor Lane**
**London EC2Y 9BU (GB)**

(72) Inventor: **Ball, William John**
The British Petroleum Co. Ltd. Chertsey Road
Sunbury-on-Thames Middlesex TW16 7LN (GB)
Inventor: **Cotton, Leonard**
The British Petroleum Co. Ltd. Chertsey Road
Sunbury-on-Thames Middlesex TW16 7LN (GB)
Inventor: **Stewart, David Gordon**
The British Petroleum Co. Ltd. Chertsey Road
Sunbury-on-Thames Middlesex TW16 7LN (GB)

(74) Representative: **Harry, John et al,**
c/o The British Petroleum Company plc Patents
Division Chertsey Road
Sunbury-on-Thames Middlesex TW16 7LN (GB)

(56) References cited:
synthesis of ethanol from Co and H2 under
atmospheric pressure over pyrolysed rhodium
carbonyl clusters on TiO2,ZrO2 and La2O3"
pages 566-567
PATENTS ABSTRACTS OF JAPAN, vol. 3, no.
68, June 13, 1979, page 48 C 48

The file contains technical information
submitted after the application was filed and not
included in this specification

## Description

The invention concerns the production of an oxygenated hydrocarbon product containing ethanol by reacting a mixture of carbon monoxide and hydrogen, hereinafter to be referred to as synthesis gas, at a temperature in the range from 150 to 450°C and a pressure in the range from 1 to 700 bars over a catalyst comprising a supported mixture of a rhodium component and a zirconium component.

Oxygenated hydrocarbons, and in particular $C_2$-oxygenated hydrocarbons, such as acetic acid, ethanol and acetaldehyde are valuable industrial products. On a commercial scale acetic acid is generally produced either by oxidation or paraffinic hydrocarbon fractions or by carbonylation of methanol; ethanol is produced either by fermentation of natural products, eg molasses or by hydration of ethylene in the presence of an acid catalyst and acetaldehyde is produced by the Wacker process. The dwindling reserves of crude oil from which many of the above feedstocks are derived and the associated need to utilise fully the remaining natural resources such as coal and the vast amounts of gases, eg methane potentially available from the exploitation of North Sea oilfields, has stimulated research into the utilisation of synthesis gas which can readily be obtained not only from crude oil but also from both coal and methane gas. Much of the early work on synthesis gas conversion involved the use as catalysts of the iron group metals, ruthenium and various metal oxide systems. One general disadvantage of such systems is that catalysts which possess acceptable activity generally tend to be unselective ie they produce a wide spectrum of products including both hydrocarbons and oxygenated hydrocarbons having a broad distribution of carbon numbers. This not only complicates the recovery of the desired products but also results in the wastage of reactants to undesirable products. On the other hand those catalysts having acceptable selectivity generally have a low activity thereby necessitating recycle of large quantities of unchanged reactants.

In US Patent No 4246186 (Union Carbide Corp) there is disclosed a process which, it is claimed, overcomes the aforesaid disadvantage of the prior art processes. The process for selectively producing $C_2$-oxygenated hydrocarbons involves continuously contacting synthesis gas with a heterogeneous catalyst essentially comprising rhodium metal under reaction conditions correlated so as to favor the formation of a substantial proportion of acetic acid, ethanol and/or acetaldehyde. Subsequent patent applications describe the production of ethanol by contacting synthesis gas with a rhodium/iron catalyst (US Patent No 4235801), a rhodium/manganese catalyst (United States Patent No 4014913, a rhodium/molybdenun or rhodium/tungsten catalyst (USP 4096164), a rhodium/ruthenium catalyst (USP 4101450) and a rhodium/uranium/thorium catalyst (USP 4162262).

FR—A—2234256 describes a process for the production of polyhydric alcohols, their ether and ester derivatives, oligomers of such alcohols and monohydric alcohols and their ether and ester derivatives by reacting oxides of carbon and hydrogen in the presence of a rhodium carbonyl complex supplied to the reaction as a rhodium carbonyl cluster having an infrared spectrum which exhibits three intensive wave-length bands between about plus and minus $10\ cm^{-1}$ of about $1868\ cm^{-1}$, about $1838\ cm^{-1}$ and about $1785^{-1}$.

M. Ichikawa in Journal of the Chemical Society, Chemical Communications *13*, 566—567 (1978) describes the production of $C_2$-oxygenated compounds composed mainly of ethanol from carbon monoxide and hydrogen under atmospheric pressure over pyrolysed rhodium carbonyl clusters dispersed on specific metal oxides such as $TiO_2$, $ZrO_2$ and $La_2O_3$. Also, in the Bulletin of the Chemical Society of Japan, 51/8, 2273—2277 (1978), M. Ichikawa describes the catalytic production of ethanol by reacting carbon monoxide and hydrogen at atmospheric pressure and at 150 to 250°C over rhodium crystallite catalysts prepared from rhodium carbonyl cluster compounds highly dispersed on $La_2O_3$, $TiO_2$ and $ZrO_2$.

EP—A1—0 004 656 describes a process for the production of acetic acid, ethanol, acetaldehyde and secondary products if any thereof by reacting carbon monoxide with hydrogen in the presence of rhodium metal-containing supported catalysts, wherein the catalysts contain in addition halide ions and magnesium salts and/or magnesium complex compounds and/or compounds of magnesium with oxides of elements of the IIIrd to the VIth Group of the Periodic Table.

EP—A1—0 010 295, published after the priority date claimed for this application, but claiming an earlier priority date, describes a process for the production of ethanol by reacting carbon monoxide with hydrogen over a supported rhodium catalyst, which contains one or more of the elements zirconium, hafnium, lanthanum, platinum, chromium and mercury as co-catalysts.

Our own European patent application publication No 0018763 describes the use of a rhodium/chromium catalyst for the production of $C_1$ to $C_4$ oxygenated hydrocarbons from synthesis gas.

We have now found that a catalyst comprising a supported mixture of a rhodium component, a zirconium component and one or more metal components selected from iron, manganese, molybdenum, tungsten, ruthenium, chromium, uranium, thorium, iridium and palladium is active for the selective production of an oxygenated hydrocarbon product containing ethanol.

Synthesis gas in the form of mixtures of the gases carbon monoxide and hydrogen is abundantly available. Methods for preparing synthesis gas are well-known in the art and usually involve the partial oxidation of a carbonaceous substance, eg coal. Alternatively synthesis gas may be prepared, for example, by the catalytic steam reforming of methane. Although it is preferred to use substantially pure synthesis gas the presence of such impurities as carbon dioxide and nitrogen can be tolerated. On the other hand impurities which have a deleterious effect on the reaction should be avoided. The ratio of hydrogen to carbon monoxide in the synthesis gas may vary widely. Normally the molar ratio of hydrogen to carbon monoxide may be in the range from 20:1 to 1:20, preferably from 5:1 to 1:5 and even more preferably from 1:1 to 2:1. Increasing the molar ratio of hydrogen to carbon monoxide beyond 2:1 generally tends to increase the total rate of reaction but only at the expense of an increase in selectivity to methane. Increasing the molar ratio of hydrogen to carbon monoxide also favours the formation of more highly reduced oxygenated products. Thus, as the ratio increases so the formation of ethanol, rather than acetaldehyde or acetic acid, is favoured. Methods for adjusting the molar ratio by the so-called 'shift reaction' are well-known to those versed in the art.

The catalyst comprises a supported mixture of a rhodium component, a zirconium component and one or more metal components selected from iron, molybdenum, tungsten, ruthenium, chromium, uranium, thorium, iridium and palladium. A wide variety of support materials may be employed. Suitable support materials include silica, alumina, silica/alumina, magnesia, thoria, titania, chromia, zirconia and active carbon, of which silica is preferred. Zeolite molecular sieves and in particular the crystalline zeolites may also be employed. Suitably the support has a relatively high surface area. The support may have a surface area up to 350 square metres per gram (BET low temperature nitrogen adsorption isotherm method). Preferably in the range 1 to 300 square metres per gram. Whilst the actual form of the rhodium, zirconium and cocatalyst components under the reaction conditions is not known with any degree of certainty it is probable that they are either in the oxide form or the metallic form. Under the reducing conditions prevailing it is more likely that the components are in the metallic form. Thus the components may be added in the form of the metals themselves or in the form of metal compounds and may be added concurrently or sequentially. The components may be deposited on the support by any of the techniques commonly used for catalyst preparation. Although it is possible to add particles to the metals to the support it is preferred to use the techniques of impregnation from an organic or inorganic solution, precipitation, coprecipitation or cation exchange. Conveniently the catalyst may be prepared by impregnating the support with a solution or solutions of a rhodium, a zirconium and co-catalyst metal compound which may be organic or inorganic compounds. Suitable compounds are the salts of the metals eg the halides, particularly the chlorides and nitrates. Following impregnation the catalyst is preferably dried and calcined. The amount of each of the rhodium component and the zirconium component on the support may suitably be in the range of from 0.01 to 25 weight per cent, preferably from 0.1 to 10 weight per cent, of each metal based on the combined weight of the metals and the support. The, or each, additional metal component may suitably be present in any amount in the range from 0.1 to 25 weight per cent based on the combined weight of the metals and the support.

The support may also be activated by the addition of a suitable metal or non-metal component prior to incorporation of the rhodium and zirconium components. Whilst a wide variety of such metals and non-metals may be added, the alkali metals, thorium, manganese, rhodium, iron, chromium, molybdenum, boron and phosphorus are specific examples of materials which may be used. Any of the know techniques for catalyst preparation hereinafter referred to may be used for addition of the activating material. In the case of a metal activator the support is preferably impregnated with a solution of a compound of the metal, suitably the nitrate or chloride, and is thereafter dried, suitably by evaporation and calcined. The activated support is then in a suitable condition for addition of the rhodium and zirconium components and other metal components. The amount of activator added may suitably be in the range 0.01 to 50 weight percent, preferably from 1 to 25 weight percent, of the activator element based on the combined weight of the activator element and the support.

The reaction conditions which result in the formation of an oxygenated hydrocarbon product containing ethanol are suitably a temperature in the range from 150 to 450°C, preferably from 200 to 400°C, and even more preferably from 200 to 300°C. The use of higher temperatures within the aforesaid range tends to increase the co-production of methane. Because of the highly exothermic nature of the reaction the temperature requires careful control in order to prevent a runaway methanation, in which methane formation increases with increasing temperature and the resulting exotherm increases the temperature still further. In fixed bed operations, temperature control may be achieved by mixing the catalyst with an inert diluent thereby ensuring that the exothermic heat is more evenly distributed. In this way the catalyst may be protected and its useful life prolonged. Reaction pressure is suitably in the range from 1 to 700 bars, preferably from 20 to 300 bars. The use of higher pressures within the aforesaid ranges increases the production rate of $C_2$-oxygenated hydrocarbons.

An important reaction parameter is the conversion. A low conversion, preferably less than 20% of the carbon monoxide favours the formation of acetic acid, ethanol and/or acetaldehyde. A low conversion may suitably be achieved in a continuous process by employing a high space velocity. Suitably the gas hourly space velocity (volume of synthesis gas, at STP, per volume of catalyst per hour) is greater than $10^3$ per hour; preferably the gas hourly space velocity is in the range from $10^4$ to $10^6$ per hour. Excessively high space velocities result in an uneconomically low conversion while excessively low space velocities result in a loss of selectivity to desirable products.

Although the reaction may be carried out batchwise it is preferably carried out in a continuous manner.

The catalyst may be employed in the form of a fixed or fluidised bed. The desired oxygenated products may be recovered from the effluent from the reaction by various means, such as scrubbing and/or distillation. The residual gas which consists mainly of unreacted synthesis gas may be mixed with fresh carbon monoxide and hydrogen to give the required feed ratio and this composite gas then recycled to the reaction.

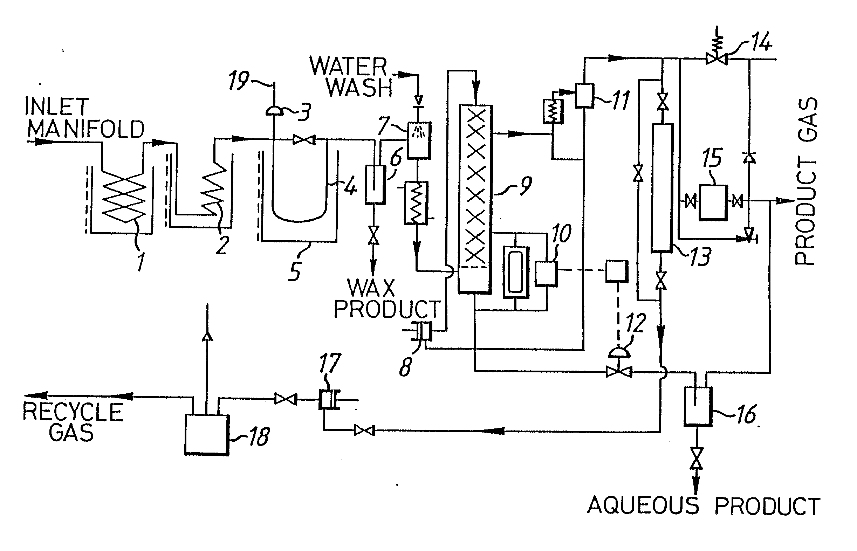

The process of the invention will now be illustrated by the following Examples and by reference to the accompanying Figure which is a simplified flow diagram of the apparatus employed.

With reference to the Figure, 1 is preheater (150°C), 2 is a preheater (200°C), 3 is a bursting disc, 4 is a reactor, 5 is a salt pot, 6 is a knock-out pot, 7 is a water quench, 8 is a water recycle pump, 9 is a water wash tower, 10 is a DP level controller, 11 is a knock-out pot, 12 is a Foxboro valve, 13 is a molecular sieve drier, 14 is a Gyp relief valve, 15 is a back pressure regulator, 16 is an aqueous product receiver, 17 is a gas recycle pump, 18 is a ballast vessel and 19 is a vent.

## Catalyst Preparation

### Catalyst A
*Rhodium/Zirconium/Silica*

Rhodium trichloride trihydrate (1.3 g) and zirconium tetrachloride (1.3 g) were dissolved in deionised water (25 ml) and added to Davison grade 59 silica (10 g, 8—16 mesh granules). The mixture was evaporated to dryness on a steam-bath and dried at 120°C for 16 hours. The catalyst was then reduced in hydrogen at 450°C for 5 hours.

### Catalyst B
*Rhodium/Zirconium/Chromium/Molybdenum/Silica*

Ammonium heptamolybdate tetrahydrate (0.42 g), chromium nitrate nonahydrate (3.8 g), rhodium trichloride trihydrate (1.3 g) and zirconium tetrachloride (1.3 g) were dissolved in deionised water (30 ml) and the resulting solution added to Davison grade 59 silica (10 g, 8—16 mesh granules). The mixture was evaporated to dryness on the steam-bath and the solid dried at 120°C for 16 hours. The catalyst was reduced in hydrogen at 450°C for 3 hours.

### Catalyst C
*Rhodium/Zirconium/Molybdenum/Silica*

Ammonium heptamolybdate tetrahydrate (0.21 g), zironium tetrachloride (1.95 g) and rhodium trichloride trihydrate (1.3 g) were dissolved in deionised water (20 ml) and the resulting solution added to Davison grade 59 silica (10 g, 8—16 mesh granules). The mixture was evaporated to dryness on a steam-bath and the solid dried at 120°C for 16 hours. The catalyst was reduced in hydrogen at 450°C for 3 hours.

### Catalyst D
*Rhodium/Zirconium/Molybdenum/Silica*

Zirconium tetrachloride (1.3 g) and rhodium trichloride trihydrate (1.3 g) were dissolved in deionised water (20 ml) and added to Davison silica grade 59 (10 g, 8—16 mesh). The mixture was evaporated by dryness on a steam-bath and the solid dried at 120°C for 16 hours and reduced in hydrogen at 450°C for 3 hours. Ammonium heptamolybdate tetrahydrate (0.42 g) was dissolved in deionised water (20 ml) and the solution added to the above catalyst. The mixture was evaporated to dryness on a steam-bath, dried at 120°C for 16 hours and treated in hydrogen at 450°C for 3 hours.

### Catalyst E
*Rhodium/Zirconium/Molybdenum/H-form Aluminosilicate (Mordenite)*

Ammonium heptamolybdate tetrahydrate (0.63 g), zirconium tetrachloride (1.95 g) and rhodium trihydrate (1.95 g) were dissolved in deionised water (16 ml). Half of this solution was then added to H-mordenite (15 g, 1/16 in extrudate) and the mixture dried at 100°C for one hour. The remaining solution was added and the whole evaporated to dryness on a steam-bath and dried at 120°C for 16 hours. The catalyst was reduced in hydrogen at 450°C for 2 hours.

### Catalyst F
*Rhodium/Zirconium/Manganese/Silica*

Manganous acetate tetrahydrate (0.2 g), zirconium tetrachloride (1.3 g) and rhodium trichloride trihydrate (1.3 g) were dissolved in deionised water (20 ml) and the resulting solution added to Davison silica grade 59 (10 g, 8—16 mesh granules). The mixture was evaporated to dryness on a steam-bath and the solid dried at 120°C for 16 hours and reduced in hydrogen at 450°C for 2 hours.

Catalyst G
*Rhodium/Zirconium/Palladium/Silica*

Palladium dichloride (0.2 g), zirconium tetrachloride (1.3 g) and rhodium trichloride trihydrate (1.3 g) were dissolved in deionised water (20 ml) and the resulting solution was added to Davison silica, grade 59 (10 g, 8—16 mesh). The mixture was evaporated to dryness of a steam-bath and the solid dried at 120°C for 16 hours. The catalyst was reduced in hydrogen at 450°C for 2 hours.

Catalyst H
*Rhodium/Zirconium/Iridium/Silica*

Ammonium chloroiridate (0.2 g), zirconium tetrachloride (1.3 g) and rhodium trichloride trihydrate (1.3 g) were dissolved in deionised water (20 ml) and the resulting solution added to Davison silica, grade 59 (10 g, 8—16 mesh). The mixture was evaporated to dryness on a steam-bath and the solid dried at 120°C for 16 hours. The catalyst was reduced in hydrogen at 450°C for 2 hours.

Catalyst I
*Rhodium/Zirconium/Iron/Silica*

Ferric nitrate nonahydrate (1.45 g), zirconium tetrachloride (1.3 g) and rhodium trichloride trihydrate (1.3 g) were dissolved in deionised water (20 ml) and the resulting solution added to Davison silica, grade 59 (10 g, 8—16 mesh granules). The mixture was evaporated to dryness on a steam-bath and dried at 120°C for 16 hours. The catalyst was then reduced with hydrogen at 450°C for 3 hours.

Catalyst J
*Rhodium/Zirconium/Uranium/Silica*

Uranylacetate dihydrate (0.45 g), zirconium tetrachloride (1.3 g) and rhodium trichloride trihydrate (1.3 g) were dissolved in deionised water (20 ml) and the resulting solution added to Davison silica, grade 59 (10 g, 8—16 mesh granules). The mixture was evaporated to dryness on a steam-bath and dried at 120°C for 16 hours. The catalyst was then reduced with hydrogen at 450°C for 3 hours.

Catalyst K
*Rhodium/Zirconium/Tungsten/Silica*

Ammonium metatungstate tetrahydrate (0.3 g), zirconium tetrachloride (1.3 g) and rhodium trichloride trihydrate (1.3 g) were dissolved in deionised water (20 ml) and concentrated hydrochloric acid (2 ml) and the resulting solution added to Davison silica grade 59 (10 g, 8—16 mesh granules). The mixture was evaporated to dryness on a steam-bath and dried at 120°C for 16 hours. The catalyst was then reduced at 450°C in hydrogen for 6 hours.

Catalyst L
*Rhodium/Zirconium/Thorium/Silica*

Thorium nitrate hexahydrate (5 g) was dissolved in deionised water and added to Davison silica, grade 59 (10 g, 8—16 mesh granules) and the mixture was evaporated to dryness on a steam-bath. The support was then heated at 400°C in air for 4 hours.

Zirconium tetrachloride (1.3 g) and rhodium trichloride trihydrate (1.3 g) were dissolved in deionised water (20 ml) and the resulting solution added to the above support. The mixture was evaporated to dryness of a steam-bath and dried at 120°C for 16 hours. The catalyst was reduced for 6 hours in hydrogen at 450°C.

## Synthesis gas conversion

Comparison Test

With reference to the accompanying Figure a mixture of carbon monoxide and hydrogen in a molar ratio of 1:2 was passed via the inlet manifold through the two preheater coils (1) and (2) maintained at 150°C and 200°C respectively in silicone oil baths. The heated gases were than fed via a heat-traced line to the copper-lined reactor (4) containing a fixed bed of Catalyst A in the form of 8 to 16 mesh (BSS) granules. The reactor was maintained at the desired reaction temperature by immersion in a molten salt bath (5). The product gases were passed via a heat-traced line thorugh a knock-out pot for wax products (16) into a small quench vessel (7) into the top of which water was sprayed. The gases were then passed through a water cooler to the bottom of the water wash tower (9) which was packed with 3/8 inch Raschig rings. In the tower (9) the product gases were washed counter-current with water. The resulting liquid product was fed into the receiver (16) and any dissolved gases were recombined with the product gas stream from the back pressure regulator (15). The separated gas stream from the top of the water wash tower (9) was passed through a water cooler to the knock-out pot (11) and then to the inlet side of the dome-loaded back pressure regulator (15). Recycle gas was recovered from the inlet side of the back pressure regulator (15), passed through a molecular sieve drier (13) and compressed up to 67 bars in the gas ballast vessel (18) using the gas recycle pump (17). The recycle gas was fed back to the inlet manifold. Provision was made to feed spot samples of the inlet gases and the total gas stream to a gas chromatographic analytical unit.

5

The product gas stream leaving the back pressure regulator (15) was measured and samples were withdrawn and analysed by gas chromatography. The liquid product was also sampled and analysed by gas chromatography.

After the reaction had reached equilibrium a balance run was carried out over a one hour period at a temperature of 274°C.

### Example 1

The procedure of the Comparison Test was followed using Catalyst B in place of Catalyst A. The run was carried out at 257°C instead of 274°C.

### Example 2

The procedure of the Comparison Test was followed using Catalyst C in place of Catalyst A. The run was carried out at 240°C.

### Example 3

The procedure of the Comparison Test was followed using Catalyst D in place of Catalyst A. Runs were carried out at 230°C and 240°C.

### Example 4

The procedure of the Comparison Test was followed using Catalyst E in place of Catalyst A. Runs were carried out at 250°C and 262°C.

### Example 5

The procedure of the Comparison Test was followed using Catalyst F in place of Catalyst A. Runs were carried out at 261°C and 285°C.

### Example 6

The procedure of the Comparison Test was followed using Catalyst G in place of Catalyst A. Runs were carried out at 280°C and 290°C.

### Example 7

The procedure of the Comparison Test was followed using Catalyst H in place of Catalyst A. Runs were carried out at 233°C, 280°C and 305°C.

### Example 8

The procedure of the Comparison Test was followed using Catalyst I in place of Catalyst A. The run was carried out at 273°C.

### Example 9

The procedure of the Comparison Test was followed using Catalyst J in place of Catalyst A. The run was carried out at 267°C.

### Example 10

The procedure of the Comparison Test was followed using Catalyst K in place of Catalyst A. The run was carried out at 251°C.

### Example 11

The procedure of the Comparison Test was followed using Catalyst L in place of Catalyst A. The run was carried out at 290°C.

The results of the Comparison Test and Example 1 to 11 are given in the Table.

In the Table:

1. CO Conversion $= \dfrac{\text{Moles of carbon monoxide consumed}}{\text{Moles of carbon monoxide fed}} \times 100$

2. Selectivity $= \dfrac{\text{Moles of carbon monoxide converted to particular product}}{\text{Moles of carbon monoxide consumed}}$

3. Greater than $C_2$ = Hydrocarbons with carbon numbers greater than 2

4. MeOH = Methanol

5. EtOH = Ethanol

6. Acet = Acetaldehyde

7. n-PrOH = *n*-propanol

6

TABLE

Reaction parameters: GHSV = 48,000 h—1     H$_2$ : CO Molar ratio = 2:1
Pressure = 50 bars     Recycle gas ratio = 19:1

| Example | Catalyst | Reaction Temp (°C) | CO Conversion[1] (%) | SELECTIVITY (%) [2] | | | | | | | |
|---|---|---|---|---|---|---|---|---|---|---|---|
| | | | | CO$_2$ | CH$_4$ | >C$_2$[3] | MeOH [4] | EtOH [5] | Acet [6] | n-PrOH [7] | Acetic Acid |
| Comp Test | A | 274 | 12.2 | 2.7 | 34.6 | 1.1 | 7.3 | 39.5 | 7.6 | Nil | 7.1 |
| 1 | B | 257 | 31.2 | 14.5 | 30.1 | 3.5 | 25.3 | 21.9 | Nil | 2.1 | 3.0 |
| 2 | C | 240 | 19 | 11 | 25 | 4 | 22 | 27 | Nil | 7 | 4 |
| 3 | D | 230 | 25 | 4 | 14 | 3 | 50 | 23 | Nil | 5 | 1 |
| | | 240 | 28 | 5 | 22 | 4 | 38 | 24 | Nil | 4 | 3 |
| 4 | E | 250 | 15 | 3 . | 30 | 8 | 26 | 23 | Nil | 2 | 8 |
| | | 262 | 22 | 4 | 39 | 8 | 28 | 17 | Nil | 2 | 2 |
| 5 | F | 261 | 11 | 1 | 29 | 2 | 11 | 36 | 11 | 3 | 6 |
| | | 275 | 17 | 2 | 35 | 3 | 6 | 40 | 6 | Nil | 8 |
| | | 285 | 24 | 2 | 39 | 3 | 5 | 42 | 5 | 2 | 2 |
| 6 | G | 280 | 18 | 4 | 49 | 1 | 11 | 25 | 6 | Nil | 4 |
| | | 290 | 20 | 2 | 56 | 1 | 9 | 24 | 3 | Nil | 5 |

0 030 110

TABLE (Continued)

| Example | Catalyst | Reaction Temp (°C) | CO Conversion [1] (%) | SELECTIVITY (%) [2] | | | | | | | |
|---------|----------|---------|---------|-----|-----|-----|-----|-----|-----|-----|-----|
| | | | | $CO_2$ | $CH_4$ | $C_2$ [3] | MeOH [4] | EtOH [5] | Acet [6] | n-PrOH [7] | Acetic Acid |
| 7 | H | 233 | 5 | 3 | 21 | Nil | 21 | 38 | 9 | Nil | 8 |
| | | 280 | 10 | 2 | 39 | 1 | 14 | 29 | 8 | 2 | 5 |
| | | 305 | 17 | 2 | 56 | 1 | 10 | 25 | 2 | 1 | 3 |
| 8 | I | 273 | 28 | 1 | 34 | 1 | 34 | 27 | Nil | 2 | 1 |
| 9 | J | 267 | 17 | 1 | 35 | 3 | 13 | 31 | 10 | 2 | 5 |
| 10 | K | 251 | 24 | 2 | 21 | 4 | 22 | 36 | 4 | 4 | 7 |
| 11 | L | 290 | 30 | 1 | 31 | 2 | 13 | 37 | 9 | 3 | 6 |

0 030 110

**0 030 110**

## Claims

1. A process for the production of an oxygenated hydrocarbon product containing ethanol by reacting carbon monoxide and hydrogen at a temperature in the range from 150 to 450°C and a pressure in the range from 1 to 700 bars over a catalyst comprising a supported mixture of a rhodium component and a zirconium component, characterised in that there is also present on the support one or more metal components selected from iron, manganese, molybdenum, tungsten, ruthenium, chromium, uranium, thorium, iridium and palladium.

2. A process according to claim 1 wherein the support is silica.

3. A process according to either claim 1 or claim 2 wherein the support is activated by the addition of an alkali metal, thorium, manganese, rhodium, iron, chromium, molybdenum, boron or phosphorus and calcined prior to addition of the rhodium and zirconium components.

4. A process according to any one of the preceding claims wherein the temperature is in the range from 200 to 400°C, the pressure is in the range from 20 to 300 bars and the gas hourly space velocity is in the range from $10^4$ to $10^6$ per hour.

5. A process according to any one of the preceding claims wherein the catalyst comprises a rhodium component, a zirconium component and a molybdenum component supported on silica.

## Patentansprüche

1. Verfahren zur Herstellung eines Ethanol enthaltenden, sauerstoffhaltigen Kohlenwasserstoff-produktes durch Umsetzung von Kohlenmonoxid und Wasserstoff bei einer Temperatur im Bereich von 150 bis 450°C und einem Druck im Bereich von 1 bis 700 bar über einem Katalysator, der eine auf einem Träger abgeschiedene Mischung aus einer Rhodiumkomponente und einer Zirkoniumkomponente umfaßt, dadurch gekennzeichnet, daß auf dem Träger noch eine oder mehrere Metallkomponenten, ausgewählt aus Eisen, Mangan, Molybdän, Wolfram, Ruthenium, Chrom, Uran, Thorium, Iridium und Palladium, anwesend ist bzw. sind.

2. Verfahren nach Anspruch 1, in welchem der Träger Kieselsäure ist.

3. Verfahren nach Anspruch 1 oder 2, in welchem der Träger vor der Zugabe der Rhodium- und Zirkoniumkomponenten durch die Zugabe eines Alkalimetalls, Thorium, Mangan, Rhodium, Eisen, Chrom, Molybdän, Bor oder Phosphor aktiviert und calciniert wird.

4. Verfahren nach einem der vorhergehenden Ansprüche, in welchem die Temperatur im Bereich von 200 bis 400°C, der Druck im Bereich von 20 bis 300 bar und die stündlich Raumgeschwindigkeit des Gases im Bereich von $10^4$ bis $10^6$ pro h·liegen

5. Verfahren nach einem der vorhergehenden Ansprüche, in welchem der Katalysator eine Rhodiumkomponente, eine Zirkoniumkomponente und eine Molybdänkomponente auf Kieselsäure als Träger enthält.

## Revendications

1. Procédé de production d'un produit hydrocarboné oxygéné contenant de l'éthanol, par réaction du monoxyde de carbone et de l'hydrogène à une température comprise entre 150 et 450°C et à une pression comprise entre 1 et 700 bars, sur un catalyseur comprenant un mélange, sur support, d'un constituant à base de rhodium et d'un constituant à base de zirconium, procédé caractérisé en ce qu'il y a également présence, sur le support, d'un ou plusieurs constituants à base de métal, choisis parmi le fer, le manganèse, le molybdène, le tungstène, le ruthénium, le chrome, l'uranium, le thorium, l'iridium et le palladium.

2. Procédé selon la revendication 1, dans lequel le support est de la silice.

3. Procédé selon la revendication 1 ou la revendication 2, dans lequel le support est activé par l'addition d'un métal alcalin, de thorium, de manganèse, de rhodium, de fer, de chrome, de molybdène, de bore ou de phosphore, et il est calciné avant l'addition des constituants à base de rhodium et de zirconium.

4. Procédé selon l'une quelconque des revendications précédentes, dans lequel la température se situe entre 200 et 400°C, la pression se situe entre 20 et 300 bars, et la vitesse spatiale horaire de gaz se situe entre $10^4$ et $10^6$ par heure.

5. Procédé selon l'une quelconque des revendications précédentes, dans lequel le catalyseur comprend un constituant à base de rhodium, un constituant à base de zirconium et un constituant à base de molybdène, sur un support de silice.